Europäisches Patentamt

European Patent Office

Office européen des brevets

Publication number: **0 299 376**
**A2**

# EUROPEAN PATENT APPLICATION

Application number: **88110916.9**

Date of filing: **08.07.88**

Int. Cl.⁴: **A61K 45/06 , A61K 37/54 ,
A61K 31/725 , A61K 31/557 ,
//(A61K37/54,31:725,31:557)**

Priority: **13.07.87 US 72461**

Date of publication of application:
**18.01.89 Bulletin 89/03**

.Designated Contracting States:
**AT BE CH DE ES FR GB IT LI NL SE**

Applicant: **Shell, William Elson**
**956 Chantilly Lane**
**Los Angeles California 90277(US)**

Applicant: **See, Jackie R.**
**541 Riviera Court**
**Fullerton California 92635(US)**

Inventor: **Shell, William Elson**
**956 Chantilly Lane**
**Los Angeles California 90277(US)**
Inventor: **See, Jackie R.**
**541 Riviera Court**
**Fullerton California 92635(US)**

Representative: **LOUIS, PÖHLAU, LOHRENTZ &
SEGETH**
**Kesslerplatz 1 Postfach 3055**
**D-8500 Nürnberg(DE)**

Prostanoid compositions and methods for reducing blood vessel dysfunction.

Prostanoid compositions and methods for reducing dysfunction in human blood vessels, particularly, as a result of mechanical intervention in a blood vessel such as by a transluminal angioplasty procedure, or as a result of unstable anginal ischemia, including myocardial infarction. During acute myocardial infarction caused, e.g. by a clot in a coronary artery, a prostaglandin compounds and a thrombolytic agent are given intravenously and intra-arterially. The thrombolytic agent dissolves the clot and the prostaglandin compound provides cell protection, reduces spasm and rethrombosis due to its antiplatelet effects. In the case of a mechanical intervention as for example, in transluminal angioplasty procedures, the thrombolytic agent is given intravenously prior to the angioplasty procedure and the prostaglandin compound is administered intra-arterially during the angioplasty procedure. Further, and in a preferred embodiment, the prostaglandin compound is administered intravenously for a selected time period after the procedure. The combination is effective because the thrombolytic compounds do not affect platelet vessel wall interactions as do the prostaglandins.

## PROSTANOID COMPOSITIONS AND METHODS FOR REDUCING BLOOD VESSEL DYSFUNCTION

### BACKGROUND OF THE INVENTION

#### 1. Field of the Invention

This invention relates in general to certain new and useful improvements in methods for reducing blood vessel dysfunction in both stable and unstable angina pectoris, and more particularly, to a method of the type stated which relies upon the intravenous administration of a pharmacological amount of a thrombolytic agent, combined with intra-arterial or intravenous administration of a pharmacological amount of a prostaglandin compound, and compositions containing specified amounts of the ingredients to produce a high degree of efficacy.

#### 2. Brief Description of the Prior Art

In order to overcome some of the dysfunction resulting from percutaneous transluminal coronary angioplasty, heparin and intracoronary nitroglycerine, as well as systemic antiplatelet agents and calcium blockers have been used. In addition, various preparations have been employed prior to the angioplasty procedures and include, for example, aspirin, dysyridamole and other antiplatelet agents, intravenous dextran, and certain steroids. Nevertheless, the various complications still persist. Moreover, these complications of abrupt occulsion and early restenosis account for virtually all of the problems in percutaneous transluminal coronary angioplasty. In the NHLBI registery, as reported by Cowley et al in the American Journal of Cardiology, 1984, the results of coronary angioplasty in 3,079 patients were examined and 418 patients suffered some form of post angioplasty procedure complications, dissection or abrupt occlusion.

There have been numerous publications dealing with the various types of prostaglandin compounds and their effectiveness for providing unique antiplatelet effects and antithrombotic effects and antispasmic effects. For example, U.S. Patent No. 4,239,778 to Venton et al, discloses a novel azaprostanoic acid analog and its effectiveness as an inhibitor of platelet aggregation. U.S. Patent No. 4,095,036 to Yankee discloses an 8-beta 12-alpha PG-2 prostaglandin type analog and its effectiveness in controlling spasm, particuarly in asthmatic conditions, as well as a decrease in blood platelet adhesiveness. This patent also discloses the use of various prostaglandin analogs as being effective to prevent the formation of thrombi to thereby prevent post operative thrombosis and their effectiveness in and prevention of myocardial infarcts.

In like manner, U.S. Patent No. 4,205,178 to Axon discloses various prostaglandin E derivatives and analog compounds which are also effective in prevention of myocardial infarcts and effective in inhibiting platelet aggregation. This patent also discloses the use of these compounds as hypotensive agents when administered at a rate of about 0.01 to about 50 micrograms per kilogram of body weight per minute.

There have been several articles dealing with the effects of prostaglandin metabolism as a result of arterial insult. For example, the article entitled "Vessel Wall Arachidonate Metabolism After Angioplasty" by Andrew Cragg, M.D. et al discusses the possible mechanism of post angioplasty vasospasm and the postulation that an inadequate vessel wall production of prostaglandin I-2 or prostaglandin E-2 might contribute to spasm of a dilated artery, as reported in the May 1, 1983 edition of the American Journal of Cardiology, Volume 51, pages 1441 et seq. In like manner, paralysis and hyperemia of an arterial wall and altered vasomotor tone has been demonstrated following percutaneous transluminal angioplasty and which is believed to be due to the effect of inadequate production of beneficial prostaglandin compounds generated by the body. In particular, these effects were reported in "Prostaglandins and Angioplasty", as reported in Interventional Radiology, December 1983, page 681 et seq.

It is known that thrombolytic agents, such as, for example, tissue plasminogen activator, streptokinase and urokinase, are effective in dissolving blood clots after a serious insult to a body blood vessel. However, it has also been recognized that the use of the thrombolytic agents also presents some serious adverse side effects, such as, for example, a sudden re-occlusion from restenosis or spasm, thereby inducing abrupt closure of the insulted vessel.

Very recently, the use of a thrombloytic agent in connection with percutaneous transluminal coronary angioplasty has been reported by Reimund Erbel, M.D. et al, in the <u>Journal of American Cardiology</u>, September 1986, 485-95. The study reported in this recent publication showed that re-occlusion of the coronary artery was reduced along with an increase of coronary re-perfusion. The authors of this study, however, found that in a majority of the cases, it was necessary to combine a mechanical recanalization by angioplasty along with a streptokinase therapy for thrombolysis before re-perfusion.

## <u>BRIEF SUMMARY OF THE DISCLOSURE</u>

Generally, the present invention provides both methods and compositions for reducing blood vessel dysfunction in at least three different situations. The first of these situations involves the insult to a blood vessel by a mechanical means, such as a mechanical implement for purposes of e.g. for opening the blood vessel. As an example, percutaneous transluminal angioplasty procedures utilize some mechanical means for opening the blood vessel. In this situation, a thrombolytic agent is administered, usually intravenously, and usually prior to any such procedure, and the prostaglandin compound is administered intra-arterially either before or during the procedure, or both. Moreover, the prostaglandin compound may be administered after the procedure intravenously for a selected time period.

The second situation in which the methods and compositions of the present invention are useful in an unstable anginal inschemia, including but not limited to, an acute myocardial infarction, either during the infarction or shortly thereafter. In this case, a thrombolytic agent is used to open the artery, and this may be followed by the administration of a prostaglandin compound in the manner as previously described. While the prostaglandin compound does not necessarily open a blood vessel, thrombolytic agents will perform that function and the prostaglandin compound has shown the effects of maintaining the blood vessel in the opened condition.

A third situation in which the compositions and methods of the present invention are useful arise when a blood vessel is insulted by a mechanical means, usually percutaneous transluminal coronary angioplasty procedures during an acute myocardial infarction. In this case, the prostaglandin compound may be used alone or in combination with the thrombolytic agent, as aforesaid.

The prostaglandin compound is administered in relation to an artery in which an angioplasty procedure is to occur, a selected amount of intra-arterial prostaglandin compound which may be followed by an intravenous infusion. This prostaglandin compound is administered in a proper amount which will provide cell protection and provide antithrombotic effects, antiplatelet effects and antispasmic effects.

The prostaglandin compound may adopt the form of several well known prostaglandin analogs and isomers, as hereinafter described, and include, for example, the prostaglandin E compounds, the prostaglandin I compounds, the prostaglandin D compounds, the prostaglandin F compounds, Ciprostene, etc. It is well known that the normal body artery will generate one or more of the selected prostaglandin compounds, as for example, prostaglandin I-2 when the artery is insulted, that is stressed or otherwise subjected to an injury. See for example, "Vessel Wall Archiondonate Metabolism After Angioplasty" by Andrew Cragg, M.D. et al, supra. See also, "The Treatment of Vasospastic Disease With Prostaglandin E-I", British Medical Journal, Volume 201. In a typical coronary artery insult, the coronary artery will generate prostaglandin usually in an amount of about one picogram up to a maximum of about three picograms. See for example, "Vascular Prostaglandin and Thromboxane Production in a Canine Model of Myocardial Ischemia" by James M. Schmitz et al, Circulation Research, Volume 57, No. 2, August 1985; "Prostaglandins in Cardiovascular Medicine: Part 1", by John G. Harold, M.D., William E. Shell, M.D. et al; "Cardiovascular Reviews and Reports", Volume 5, No. 9, September 1984.

The term "prostaglandin compound" as used herein, generally refers to any of the prostanoid compounds including those eicosanoids which exhibit biological activity and including, for example, the cylical prostaglandin compounds. Thus, the terms "prostaglandin" and "prostaglandin compound", are used in a generic sense to encompass all such prostanoids.

Inasmuch as the prostaglandin compounds are administered in accordance with the present invention in nanogram amounts (1-billionth of a gram) and at minimum, 25 nanograms, and the amount of prostaglandin which may be generated by any body artery on insult is no greater than picogram amounts (1 trillionth of a gram) and usually less than 3 picograms, it can be seen that the prostaglandin compounds administered in accordance with the present invention are administered in amounts almost one thousand times, and usually much greater than one thousand times the amount of prostaglandin which could be generated by any normal body artery when insulted. Thus, the administration of prostaglandin compounds in the nanogram range as specified herein constitutes a pharmacological amount and does not merely operate as a replacement, as for example, in replacement therapy, that is, administration of an amount to replace that which may have been lost. As an example, cortisone may be administered when body cortisone generation is depleted, although in amounts massively greater than the amounts depleted and is thus considered to be a pharmacological administration.

In view of the above explanation of administered prostaglandin amounts, the term "pharmacological amount" is used herein to mean the administration of the prostaglandin compound in an amount substantially greater than the amount of prostaglandin which would be generated by a coronary artery when insulted, and usually in an amount of about at least one thousand times greater than the amount which would be generated by a normal coronary artery when insulted. The term "pharmacological amount" is also used herein to mean the administration of the thrombolytic agent in the range which is effective to activate clot dissolving mechanisms.

The administration of the prostaglandin along with the thrombolytic agent during acute myocardial infarction and other unstable ischemic episodes, produces unexpected results in that there has been found to be a substantially reduced incidence of blood clot reformation with the single administration of the thrombolytic agent. In addition, the administration of the prostaglandin compound and the thrombolytic agent may occur during a transluminal coronary angioplasty procedure which takes place during a myocardial infarction. In each case, the combination has been found to reduce the problems previously encountered by the administration of a thrombolytic agent alone and to reduce the effects of a mechanical canalization.

When the thrombolytic agent is used in combination with the administration of the prostaglandin compound, as described herein, there has been an observable marked decrease in the re-occlusion induced by spasm which was oftentimes found to result after an administration of a thrombolytic agent. The exact mechanism of the cooperation of the thrombolytic agent and the prostaglandin compound is not fully understood, although the prostaglandin compound does, in fact, appear to eliminate the above described undesirable side effects of the thrombolytic agent.

The administration of the prostaglandin compound in the pharmacological amount produces unexpected results in that the coronary artery or other artery in which the prostaglandin was administered remains dilated for a period of two to three times longer than if nitroglycerine or verapamil or both were administered. Moreover, the amount of arterial dilation is substantially greater and in many cases, the duct of the artery will remain dilated to twice its original size. As an example, in an angioplasty procedure, the artery may remain dilated for a period of about 45 seconds after removal of the catheter tip. With adminsitration of nitroglycerine, the artery may remain dilated for a period of about two minutes. With the administration of prostaglandin E-1, the same artery will remain dilated for a period of seven minutes or longer. This dilation period enables the body to overcome the results of abrupt occulsion or other dysfunction which often results after an angioplasty procedure. In addition, there is likely a substantially greater length of the myocardial artery which is dilated after the administration of the prostaglandin compound in the pharmacological amount.


## DETAILED DESCRIPTION OF PREFERRED EMBODIMENTS


As indicated previously, the present invention utilizes both methods and compositions to reduce blood vessel dysfunction as a result of both mechanical canalizations of blood vessels, as for example, coronary transluminal angioplasty procedures, or by any other mechanical means and is also effective in reducing dysfunction resulting from unstable ischemic epoisodes including but not limited to, acute myocardial infarction. For example, the compositions and methods of the present invention are also useful for treating the recently recognized syndrome of silent myocardial ischemia. Thus, the compositions and methods are effective in essentially treating most forms of unstable anginal ischemia.

The compositions and methods of the present invention utilizing either individually the prostaglandin compounds, or the prostaglandin compounds in combination with the thrombolytic agent are described hereinafter in connection with acute myocardial infarction. However, it should be understood that the compositions and methods are useful in treating any of the unstable anginal ischemia conditions. Moreover, the compositions and methods are described in detail hereinafter in terms of percutaneous transluminal coronary angioplasty procedures. However, and here again, it should be understood that the compositions and methods are useful in treating any canalization by mechanical means or otherwise, and for that matter, for treating any insult to a blood vessel. Therefore the following detailed description is only exemplary of the many uses of the compositions and methods of the present invention.

In accordance with the present invention, a thrombolytic agent such as a streptokinase, or urokinase, or a tissue plasminogen activator are intravenously administered to a patient with an unstagle anginal ischemia, as for example, an acute myocardial infarction, or prior to an angioplasty procedure. A prostaglandin compound, as hereinafter described in more detail, is also administered to a patient in which an anioplasty procedure is to occur. While the thrombolytic agent is administered to the patient prior to the angioplasty procedure, the prostaglandin compound may be administered prior to or during the angioplasty procedure, and for that matter, preferably after the angioplasty procedure.

The method and the compositions, as hereinafter described, in accordance with the present invention, utilize prostaglandin compounds and thrombolytic agents in combination, in one situation, to reduce dysfunction arising from angioplasty procedures when the patient is in a stable condition. The method and the compositions may also be used in connection with angioplasty procedures either during or following an acute myocardial infarction or other unstable ischemic episodes. In a third mode, the thrombolysis may be followed by the angioplasty procedure and in which only the prostgalandin compounds are used during an unstable ischemic episode. Finally, the present invention also provides only for the use of the thrombolytic compound only during an unstable ischemic episode.

In the situation where the angioplasty procedure is used with a stable patient, or during, or following an unstable ischemic episode, the thrombolytic agent is usually administered prior to any angioplasty procedure and followed by the administration of the prostaglandin compound. The thrombolytic agent may be either intravenously adminstered or intra-arterially administered and may also be administered directly to the site in which an angioplasty procedure is to occur. This may be followed by an intra-arterial adminstration of the prostaglandin compound, again to the site in which the angioplasty procedure is to occur. Finally, and in some cases, it is also desireable to intravenously administer the prostaglandin compound for a period of time after the angioplasty procedure occurs, as hereinafter described in more detail. In another mode of use, the thrombolytic agent may be used during any unstable ischemic episode followed by only an intravenous administration of a prostaglandin compound. Otherwise, the thrombolytic agent may be followed by an angioplasty procedure during an unstable ischemic episode and only with an intravenous administration of the prostaglandin compound. In other words, the bolus injections of the prostaglandin compound can be eliminated and only the intravenous adminstration for a selected time period is used.

In the post preferred embodiment, it is desirable to administer the prostaglandin compound and the thrombolytic agent during any unstable ischemia episode, or prior to any angioplasty procedure. Thereafter, the prostaglandin compound alone may be administered during and after the unstable ischemic episode. In like manner, the prostaglandin compound is administered during the angioplasty procedure. The prostaglandin compound is usually intravenously administered after the angioplasty procedure for a selected time period, as hereafter described. The prostaglandin is also preferably administered to the artery of the patient in which the angioplasty procedure is to occur.

The thrombolytic agent is administered in an amount sufficient to substantially reduce blood clot formation. The prostaglandin compound is administered in an amount required to provide cell protection, antithrombotic effects, antiplatelet effects and antispasmic effects.

The thrombolytic agents which are preferably employed in connection with the present invention are usually kinase enzymes as well as the tissue plasminogen activators. The kinase enzymes are preferably selected from the class consisting of streptokinase and urokinase. The tissue plasminogen activator is a recombinant activator ofter known as "r-TPA". These thrombolytic agents are specific in dissolving the fibrin portion of any potential blood clot and are thereby effective in reducing the formation of blood clots and dissolving any blood clot formations.

Thromboxane and leukotriene are often released from activated platelets and can result in both spasm and an abrupt closure of a coronary vessel along with early restenosis. The thrombolytic agent gives rise to the generation of the leukotriene and thromboxane. However, it has been found that the prostaglandin compound, as hereinafter described, does, in fact, inhibit the generation and activity of both the leukotriene and the thromboxane. Thus, the thrombolysin and the prostaglandin do cooperate in order to effectively reduce clot formation, as well as to provide the cell protection and the anti-thrombotic effects, anti-platelet effects, and the anti-spasmic effects.

The enzymes, such as the streptokinase and urokinase are often pretreated in order to make them effective thrombolytic agents. One of the effective treatments is to acetylate the enzymes in order to add an additional carboxyl moiety. Moreover, the enzymes can be fluorinated.

The streptokinase constitutes a sterile, purified preparation of a bacterial protein, primarily a group "CB" hemolytic streptococci. This hemolytic streptococci is generally available as a white powder containing about 25 milligrams of cross-linked gelatin polypeptides, about 25 milligrams of L-glutamate, and about 100 milligrams of normal serum albumin adapted for intravenous and intracoronary administration. Compositions of this type are made commerically available by the Hoeschst Company.

The kinase enzyme, such as the streptokinase or the urokinase, acts with a plasminogen to produce an "activator complex" which converts the plasminogen to a proteolytic enzyme plasmin. This plasmin is effective to degrade and dissolve a fibrin clot as well as fribinogen and other plasma type proteins.

It is known that when a blood clot is formed, the blood supply to a portion of the heart muscle can be reduced, if not completely cut-off. The heart muscle will be irrevocably damaged within 4 to 12 hours if there is not restoration of the blood flow. The thrombloytic agent will dissolve the blood clot effectively and thereby permit a restoration of blood supply. However, where the thrombolytic agent is used alone, in over 90% of the cases, there is still a blockage of the artery from stenosis. The prostaglandin compound is effective to reduce the dysfunction of platelet activation and vessel wall denudation which would otherwise be caused by the use of the thrombolytic agent and thereby substantially reduces the potential jeopardy to the patient as a result of the administration of the thrombolytic agent. Furthermore, there has been little occurrance of heart rhythm disturbance as a result of the administration of the thrombolytic agent in combination with the protaglandin compound.

In accordance with the present invention, and by using a combination of the thrombolytic agent along with the prostaglandin compound, there is substantial reduction of the no reflow phenomenon which has been observed with thrombolysis administration alone. The no reflow phenomenon normally occurs when a muscle cell is blood starved and thereafter there is a sudden release of blood flow to the cell. This condition can result in hemorrhage in the cell itself, or in the intercellular space. The prostaglandin compound is believed to inhibit the no reflow phenomenon by inhibiting free radical scavengers and stabilizing the cell membrane.

The thrombolytic agent and particulary the enzyme thrombolytic agents, may be reconstituted and diluted before use. Generally, the thrombolytic agent, such as streptokinase, is added to a suitable carrier such as a 5% normal sodium chloride (saline) carrier or a 5% dextrose solution. The thrombolytic agent is mixed gently with the carrier in order to reconstitute the agent. However, it is possible to reconstitute the thrombolysin in a solution normally used for its administration. After reconstitution, the solution may then be further diluted to a total volume of about 45 cc. However, the total volume could be increased to a maximum of about 500 cc. if desired.

The thrombolysin should be used within a few hours after reconsitution. However, the thrombolytic agent may be stored at about 2 to 4 degrees C. for no longer than about 24 hours and thereafter must be discarded.

The thrombolysin compound is preferably administered in an amount of no less than about 50,000 units and no more than about 3,000,000 units. This amount of thrombolysin compound will generally correspond to about 10 to about 150 milligrams. Moreover, the thrombolytic agent is usually administered by a single bolus injection.

The thrombolytic agent may also be administered by intravenous infusion over a period of time prior to the angioplasty procedure instead of by bolus injection, or in addition to a bolus injection.

The intravenous infusion of e.g. streptokinase is typically followed by an increased fibrinolytic activity. However, this activity will generally disappear within a few hours after discontinuation of the intravenous infusion of the thrombolytic agent. Nevertheless, a prolonged thrombin time may well exist for a period of up to 24 hours due to a decrease in plasma levels of fibrinogen and an increase of circulating fibrin degradation products. This thrombrin time will usually decrease to less than two times the normal amount within about 4 hours. Accordingly, the patient may be subjected to substantial bleeding problems as reported by Saul Sherry, et al, "Thrombolytic Therapy in Thrombosis", A National Institute of Health Consensus Development Conference, ANN Internal Medicine 93:141-4, 1980.

As indicated previously, the thrombolytic agent may be injected by a bolus injection or it may be administered by IV infusion over a period of time. In a combination administration, an intracoronary bolus injection of e.g. 20,000 units is followed by an intravenous administration of about 250,000 units in about 500 cc of normal saline solution or 5% glucose solution. The intravenous infusion is usually administered at a rate of about 10 cc per hour. For intravenous infusion, one vial constituting about 250,000 units may also be infused over about a 30 minute period. The intravenous infusion rate normally ranges from about 5 to about 20 cc per hour and the administration time generally ranges from about 20 minutes to about 2 hours. Higher dosages of about 3,000,000 units may also be given if desired, although this typically is not necessary.

Generally, the prostaglandin compound is administered in the region in which the balloon catheter is to be inserted in order to reduce the otherwise adverse side affects and dysfunctions which may result when attempting to dilate an artery. While the prostaglandin compound is generally administered just prior to the

6

angioplasty procedure, in many cases it is desirable to administer the prostaglandin compound to the patient during the procedure and/or for some time period thereafter and which is hereinafter described in more detail.

The method of the present invention was developed and has been proven to be highly effective in coronary angioplasty procedures. However, the method of the present invention utilizing the administration of the thrombolysin and the prostaglandin compound is also highly effective for conducting angioplasty procedures in other portions of the human body. Thus, and while the preferred embodiment of the invention deals with myocardial angioplasty procedures, it is to be understood that the invention is applicable to other angioplasty procedures as well.

These prostaglandins, which are collectively often referred to as "eicosanoids", are known to exhibit biological activitities. These activities include effects on the smooth muscles of vessels, and or inflammatory response, thermo-regulation, platelet aggregation and the like. However, heretofore, it has not been recognized that the prostaglandin compound could be effective in reducing and even overcoming most of the dysfunction resulting from and in angioplasty procedures.

Prostaglandin E-1 is one of the preferred prostaglandin compounds which may be used in accordance with the method of the present invention. The prostaglandin E-1 compound is metabolically derived from the polyunsaturated fatty acid, dihomo-y-linolenic acid. This protaglandin E-1, has the empirical formula $C_{20}H_{34}O_5$ and this prostaglandin E-1 compound has the chemical formula (11a,13E,15S)-11,15-dihydroxy-9-oxoprost-13-en-1-oic acid. Prostaglandin compounds of this type are more fully described in U.S. Patent No. 3,069,322 to Bergstrom et al.

The prostaglandin used in accordance with the present invention is not limited to the prostaglandin E-1 compound but encompasses other derivatives of prostanoic acid and includes all other prostaglandins which provide the desired pharmacological effects such as cell protection. Those prostaglandins include, for example, prostaglandin E-2 and prostaglandin E-3. The prostaglandin compounds are also deemed to include their lower alkyl esters and salts and amides which exhibit the desired pharmacological activity. The prostaglandin E-2 compounds are disclosed in U.S. Patent No. 3,598,858 and esters thereof are disclosed in U.S. Patent No. 3,691,216 and U.S. Patent No. 3,795,697.

The compositions used in the method of the present invention also include the various isomers, as aforesaid, and include for example prostaglandin A isomers and the prostaglandin B isomers. The prostaglandin A compounds do not include the hydroxyl group at carbon No. 11, but have a double bond within the ring between carbon No. 10 and carbon No. 11.

The compound prostaglandin F is also, for example, encompassed by the present invention. The prostaglandin F compounds have a hydroxyl group at the No. 9 carbon atom in place of a ketone group.

Those prostaglandin compounds having the chemical name "Ciprostene", namely, 9-methylcarbacyclin-calcuim salt and having the empirical formula $C_{20}H_{34}O_5$ are also prostaglandin compounds which are encompassed by and achieve the efficacious results in accordance with the present invention. The Ciprostene form of prostaglandin is usually characterized as a calcuim salt and is a chemically stable analog of prostacyclin.

It can be observed that the above are a non-limiting list of the prostaglandins which can be used and show that essentially any prostaglandin which provides the desired pharmacological effect can be employed. Moreover, in a broad sense and considering the generalized structural formula set forth above, it should be understood that the orientation, as shown in dotted lines, may be in or out of the plane of the cyclopentane, that is in or out of the plane of this paper.

Any carrier which is pharmaceutically acceptable and is pharmacologically inactive may be used to deliver the prostaglandin compound. Generally, dehydrated alcohols such as ethyl alcohol, normal propyl alcohol, isopropyl alcohol, etc. may be employed. Other carriers which may be employed are for example saline solutions. The amount of carrier used to deliver a given of prostaglandin is determined by the duration of the prostaglandin administration. Thus, if it is desired to administer the prostaglandin over a nine hour period, a determination of the amount of prostaglandin is made taking into consideration the patients weight, as hereinafter described. The amount of carrier selected will be sufficient to administer that determined amount of prostaglandin over the selected time period

In use, the prostaglandin compound can be stored in unopened amplules and may be stable for up to two years in a refrigerated condition. When the prostaglandin compound is dissolved in a saline solution or glucose to form an infusion solution, this infusion solution can be stable for up to about 24 hours.

The prostaglandin is administered to the region in which an angioplasty procedure is to take place in an amount of about 25 nanograms to about 3,000 nanograms and preferably about 80 nanograms to about 260 nanograms and even more preferably in an amount of about 130 nanograms, based on prostaglandin E-1, for bolus injections. In some cases, it is desirable to intravenously administer the prostaglandin compound after the angioplasty procedure or during and after an unstable ischemia episode. as for example. generally continuously for a period after the procedure, and which may vary somewhere between about 9 to about 48 hours and usually about 12 hours. In this case. the prostaglandin compound is administered in an amount of about 10 to about 100 nanograms per kilogram of body weight per minute after the procedure. based on prostaglandin E-1, and during the 9 to 48 hour period.

More preferably, the prostaglandin compound is intravenously administered in an amount of about 15 to about 40 nanograms per kilogram of body weight per minute after the procedure. based on prostaglandin E-1. and during the 9 to 48 hour post angioplasty period. The 48 hour infusing being preferred after a peripheral artery dilation, such as a femroal or iliac artery. Here again, the amounts which are administered will vary from patient to patient, depending on those patient factors mentioned above. e.g. health. age. coronory health history, reaction to administration. etc.

The present invention also provides compositions which are effective for reducing dysfunction arising out of unstable anginal ischemia and blood vessel insult, such as in angioplasty procedures and which involve the use of the prostaglandin compounds. The compositions generally comprise a carrier which contains a selected amount of the prostaglandin. The carrier must be one which does not alter the prostaglandin compound and does not inhibit its effectiveness. The carrier must also release the prostaglandin compound at a rate sufficient to dilate the blood vessels. Naturally, the carrier should be in a form which is capable of being intravenously introduced.

Each of the other prostaglandin compounds which are encompassed by the present invention have an efficacy directly related to that of prostaglandin E-1. Thus, the amounts of the other prostaglandin compounds which may be employed in the present invention is based upon the efficacy of such prostaglandin compounds compared to the efficacy of prostaglandin E-1. For this purpose, the amount of any prostaglandin compound which is used in a carrier is present in an amount necessary to produce an efficacy approximately equivalent to the efficacy produced by at least a minimum of 25 nanograms of prostaglandin E-1 to the efficacy produced by a maximum amount of about 3,000 nanograms of prostaglandin E-1. nanograms of prostaglandin E-1 to the efficacy produced by a maximum amount of about 3,000 nanograms of prostaglandin E-1.

The efficacy which is produced by any other prostaglandin compound encompassed by the present invention can be easily related to the efficacy produced by prostaglandin E-1 based on trial techniques. In other words. it is relatively simple to determine the efficacy produced by using, for example. prostaglandin I-2 and to determine the efficacy produced by using prostaglandin E-1 such that the skilled artisan can easily determine the amounts of any other prostaglandin to be used based on the use of prostaglandin E-1. It should be understood that the efficacy which can be achieved by the prostaglandin compounds will naturally vary from patient to patient, although the beneficial results which have been achieved by use of the method and the compositions of the present invention are easily noticeable. Thus, the efficacy of a given prostaglandin compound cannot be precisely determined with respect to prostaglandin E-1. but is capable of being approximately equivalently determined taking into consideration the various divergences which will occur from patient to patient.

In the case of the prostaglandin I-2 compound. this prostaglandin compound would be present in the composition administered to the patient immediately before and immediately after the angioplasty procedure in a total amount of about 3.7 nanograms to about 560 nanograms. The prostaglandin I-2 more preferably would be administered in a range of about 12 anograms to about 49 nanograms. The ciprostene prostaglandin compound would be present in the composition and administered to the patient immediately before and after the angioplasty procedure in a total account of about 187 nanograms to about 45,000 nanograms. The ciprostene prostaglandin preferably would be administered in a range of about 600 nanograms to about 3900 nanograms, and even more preferably about 1430 nanograms.

The above described quantities of prostaglandins are all based on the use of bolus injections. The amounts specified are also based on one intracoronary bolus injection prior to the angioplasty procedure and one bolus injection after the angioplasty procedure. Thus, each injection would be based on one-half the amounts specified. If intracoronary injections are made during the procedure, the amounts administered before and after would be altered. Further, the amounts administered may vary from patient to patient for the reasons mentioned above.

There are three general groups of carriers which have been found to be highly effective for use in the compositions of the invention. These carriers include isotonic solutions as well as two forms of hypertonic solutions. One type of hypertonic solution includes the angiographic contrast materials and the second form of hypertonic solution includes the biodegradable prostaglandin carrying biodegradeable microspheres.

The isotonic solutions are generally all liquid in form and are typically various salt solutions. One of the preferred isotonic solutions is a saline solution present in an amount of about 5 cc. This amount of saline is

EP 0 299 376 A2

effective to hold about 25 to about 400 nanograms of the prostaglandin E-1 compound. This amount of saline solution is also effective for generally all of the other prostaglandins encompassed by the present invention.

Other types of isotonic solutions which can be employed are 0.9 percent sodium chloride or some dextrose solutions, as for example a five percent (5%) dextrose solution. It is also possible to use a five percent (5%) dextrose solution with 0.2 percent sodium chloride therein. Lactated Ringers are also effective as one of the liquid carriers. The isotonic liquid carrier should have an osmolar strength about equivalent to that of seawater.

Angiographic contrast material can also be used as an effective carrier for the prostaglandin compounds. Many of the angiographic materials which may be used are usually X-ray absorbent as for example, hypaque-sodium. Potassium iodine and certain other recognized potassium salts may also be used as the carrier solution. It is also possible to use multiple ionic materials such as potassium iodide and iron combinations as the carrier since they all function as angiographic contrast materials.

The amount of the angiographic contrast material which is employed depends upon the amounts which are normally used to achieve the necessary contrast in an angiography procedure. Usually, about 3 cc to about 10 cc of the angiographic contrast material is used as the carrier per bolus injection.

The third group of carrier materials are the prostaglandin carrying microspheres. Generally, three major types of microspheres may be employed and these include: (1) protein laden microspheres, (2) carbohydrate laden microspheres and (3) free fatty acid laden microspheres.

The protein laden microspheres may include, for example, albumen laden microspheres. The carbohydrate laden microspheres may include, for example, various known and accepted starch laden microspheres as well as various known polysaccharides. Liposome is an effective example of a fat laden microsphere.

The term "microsphere" is used to represent particles ranging in size from about 7 u, the diameter of a red blood cell, to about 100 u in diameter. A particular group of "uniformly" sized microspheres may vary in diameter up to around 25%. Thus, a group of 10 u diameter microspheres might range in size from around 8.5 u to around 11.5 u in diameter. Usually, about 225,000 to about 500.000 microspheres are present as the carrier for a bolus injection.

The microspheres of the present invention may be composed of any long chain compound susceptible to cross linking to a solid in which amide or carboxyl groups are exposed or are capable of being exposed by suitable treatment. This includes, but is not limited to, latex materials such as polystyrene and styrene divinylbenzene, agarose, polyalkylcyanocrylate, albumin, cross-linked albumin, sucrose, starch, cellulose and dextran. Usually, about 225,000 to about 500,000 microspheres are present as the carrier for a bolus injection.

As indicated previously, the prostaglandin compound may be administered for a selected time period, as for example, nine to 48 hours after the angioplasty procedure. This intravenous administration, when coupled with the previously described intracoronary administration has been found to produce highly effective results. The amount of prostaglandin in the solution, which is administered drop-wise, should be in the range of about 10 nanograms to about 100 nanograms per kilogram of body weight per minute for prostaglandin E-1. Preferably, the prostaglandin E-1 is administered in a range of about 15 to about 40 nanograms per kilogram of body weight, per minute and even more preferably about 20 nanograms per kilogram of body weight per minute.

The amount of the other prostaglandin compounds which would be administered intravenously is also based on the efficacy of such other prostaglandins related directly to the efficacy of prostaglandin E-1. Thus, the prostaglandin in the carrier to be administered to a patient is present in an amount to deliver to the patient a desired weight of prostaglandin in nanograms per kilogram of body weight of the patient per minute to produce an efficacy equivalent to the efficacy of prostaglandin E-1 when administered in the selected range.

In a more preferred embodiment, the amount of prostaglandin E-1 is administered in a range of about 15 nanograms to about 40 nanograms per kilogram of body weight per minute, as aforesaid. With respect to the prostaglandin I-2 compound, this prostaglandin would be administered in an amount of about 1.5 nanograms to about 19 nanograms and preferably in a range of also 2 to about 7.5 nanograms per kilogram of body weight per minute. Ciprostene would be administered in an amount of about 75 nanograms to about 1500 nanograms and preferably about 112 to about 600 nanograms per kilogram of body weight per minute.

It has also been found in accordance with the present invention, that the intraveneous administration of the prostaglandin compound followed by the intracoronary administration substantially reduces the restenosis frequently encountered in angioplasty procedures. It may be observed from the following Example

9

III that there was a marked decrease in restenosis when the intraveneous administration of prostaglandin followed the intracoronary administration. In addition, when the amount of prostaglandin is reduced progressively to 0 during the last hour or two hours of the intravenous administration, there is a reduced tendency for the patient to suffer adverse effects.

The present invention is effective in providing a composition of the thrombolytic agent. The thrombolytic agent, if desired, could be pre-packaged in a single vial for ready adminstration to a patient. This is particularly desirable in the case where the prostaglandin compound is pre-packaged as hereinafter described.

The present invention is effective in providing compositions containing the above identified amounts of prostaglandin for ready administration of the same. Thus, and for example, a composition may contain a carrier and the amount of prostaglandin carried by that carrier which is necessary to produce an efficacy approximately equivalent to the efficacy produced by adminstration of prostaglandin E-1 in a range of about 25 nanograms to about 3,000 nanograms. More preferably, this composition would contain the prostaglandin compound necessary to produce an efficacy in a range approximately equivalent to the efficacy produced by the administration of 80 nanograms to about 260 nanograms of prostaglandin E-1.

The composition also preferably includes those carriers which were mentioned above and include those selected from the class consisting of (a) an isotonic solution, (b) an angiographic contrast material and (c) biodegradable prostaglandin carrying microspheres.

The present invention also provides a kit or package containing the necessary components containing the prostaglandin compound in amounts to be administered in the proper dosage. Thus, a kit may be initially provided with a first syringe containing the amount of prostaglandin compound necessary to produce an efficacy produced by administration of 12 to about 1,500 nanograms of prostaglandin E-1 and preferably the efficacy produced by the administration of about 40 to about 130 nanograms of prostaglandin E-1. The kit would also contain a second syringe for a bolus injection and which would also contain a prostaglandin compound present in an amount to produce an efficacy approximately equivalent to the efficacy of about 12 nanograms to about 1,500 nanograms of prostaglandin E-1 and preferably an efficacy approximately equivalent to that produced by the administration of about 40 nanograms to about 130 nanograms of prostaglandin E-1. Finally, this kit or package would also include a container such as a sealed package containing the amount of prostaglandin compound necessary to provide intraveneous administration for the selected time period of 9 to about 48 hours.

As a specific example of a package, the first syringe or container would contain about 65 nanograms of prostaglandin E-1 in a 1 cc saline solution and which may be diluted to about a 5 cc saline solution for use. The kit would also include a second syringe or container which contains about 65 nanograms of prostaglandin E-1 in 1 cc of a saline solution and which again may be dilluted to about a 5 cc saline solution in use. Finally, the package or kit of the present invention would include, in this example, a container of the saline solution which administers about 15 to about 40 nanograms of prostaglandin E-1 per kilogram of body weight per minute for a 12 hour period.

The thrombolytic agent can be administered as a single composition with the prostaglandin compound, or the two can be administered separately prior to the angioplasty procedure. In the example of the package using the prostaglandin compound, the first syringe or container would contain both the 65 nanograms of the prostaglandin E-1 compound as well as the desired number of units of the thrombolytic agent as for example, typically 100,000 units. Thus, the first container or syringe would contain all of the ingredients necessary to be administered pre-angioplasty in accordance with the present invention.

The prostaglandin compound, in combination with the thrombolytic agent is believed to inhibit cell necrosis by inhibiting the free radical scavengers which would be released and circulate in the blood stream. This combination is believed to stabilize the cell membrane. Other unknown factors such as ameliorating the deleterious affects of calcium in-flux into the mitochondrion are believed to also result from the use of this combination. It is further believed that the prostaglandin compound inhibits the no reflow phenomenon by inhibiting the damaging effects of eschemia, particularly along the plasma membrane. The prostaglandin compound is also believed to inhibit the free oxygen radicals which are otherwise damaging to the cells.

In patients who were studied, the peak enzyme and ECG changes were ameliorated indicating that the combination of the prostaglandin compound and thrombolytic agent does have a protective effect. In addition, it was found that further spasm or microemboli formation was inhibited, as well as the phenomenon of early restenosis.

## EXAMPLES

The invention is further described by but not limited to the following examples.

## EXAMPLE I

In order to assess the effects of intracoronary prostaglandin E-1 on myocardial blood flow in relation to a successful coronary angioplasty, twelve patients were given sixty five nanograms of the prostaglandin E-1 which was hand injected by bolus under blinded conditions. The angioplasty procedure involved a residual luminal diameter of less than 40 percent. The sixty five nanograms prostaglandin E-1 was dissolved in about four milliliters of normal saline (0.9% NaCl). Twelve additional patients were given four milliliters of normal saline as a placebo and control agent.

Digital radiographic assessments of contrast medium appearance time distal to the stenosed coronary artery under hypermic conditions were made 45 seconds before the start of the angioplasty procedure. The same radiographic assessment was made as a control under non-hypermic conditions on an intracoronary placebo 45 seconds after the conclusion of the angioplasty procedure. This information was used to measure myocardial blood flow immediately before and after the administration of the prostaglandin E-1. The known accepted method of the reciprocal of the coronary flow ratio (CFR) was used to measure myocardial blood flow. Neither the prostaglandin E-1, nor the placebo elicited any angina, dysrythmia or any significant pressure changes.

This study revealed the following data in which PGE-1 is the prostaglandin E-1 compound and PTCA represents the percutaneous transluminal coronary angioplasty procedure. N represents the number of patients, MBF represents the myocardial blood flow and CFR represents the coronary flow ratio, LAD represents the left anterior descending coronary artery and RCA represents the right coronary artery.

| Parameter | | N | LAD or RCA Stenosis | CFR |
|---|---|---|---|---|
| IC Placebo | Pre-PTCA | 6 | 80.4±11 | 1.02±0.13]p = NS |
| IC PGE-1 | Pre-PTCA | 6 | 80.4±11 | 1.14±0.17 |
| IC Placebo | Post-PTCA | 6 | 24.6±10 | 1.48±0.14]p = .001 |
| IC PGE-1 | Post-PTCA | 6 | 24.6±10 | 2.16±0.34 |

## EXAMPLE II

In order to evaluate post angioplasty effects in which prostaglandin was administered, 24 patients were selected between the ages of 18 and 75. The patients were males or otherwise post-menopausal or sterilized females. The patients undergoing the post transluminal coronary angioplasty exhibited incapacitating angina or chronic stable angina while on maximal medical therapy.

Patient Selection and Grouping

Patients who had a cerebrovascular accident or acute myocardial infarction within two months prior to the test were excluded. Also excluded were patients having unstable ventricular arrhythemia refractory to conventional anti-arrhythemic therapy. Also excluded were any patients that had cancer within the past two years unless a complete cure was clinically evident for two years. Patients with acute respiratory infections, or patients who have had surgery within several weeks preceeding the test were also excluded. Further excluded were patients that had pericardial infusion and/or severe difuse pulmonary aveolar edema, patients with previous angioplasty vessel dilation, patients with coagulation disorders, active bleeding sites or gastrointestinal bleeding, or patients with poorly controlled diabeties mellitus.

The patients were divided into three groups which included (1) a so-called "open label group". that is a group in which the patients were known to have received the prostaglandin E-1 compound. (2) a so-called "randomized, placebo group" in which the patients received a placebo compound and (3) a randomized prostaglandin E-1 compound group. The patients in this "randomized" group received compositions handed to the physicans properly marked by a pharmacist, although the identification as to which patient in the "randomized" groups (2 & 3) received the prostaglandin compound and which patient received a placebo was known only to the pharmacist and not to the physicians. (The physicians and patients were blinded: double blind trial design). The group 2 patients in the so-called "randomized placebo" group. received only a placebo composition which included only a normal saline. The group 3 patients (randomized PGE-1 group) received PGE-1 at a dose as described below.

Nine patients were included in the placebo group (group 2), eight patients were included in the drug group (group 3), and seven patients were in the open label group (group 1).

The placebo group was comprised of six men and three women. In the placebo group. there was one death within twenty four hours post-angioplasty, due to abrupt occlusion. Two of the patients in the placebo group did endure prolonged angina.

In the drug group (group 3), patients were of a similar age having an average of fifty four years and was comprised of seven men and one woman. There were not abrupt occlusions, episodes of prolonged angina or any significant complications experienced in this group.

Finally, in the open label group, five of the patients were men and two were women in a similar age range. No complications were exhibited in any patient in this post-angioplasty group.

Thus, in the three groups, the adverse event rate was 0/7 in the open label PGE-1 group, 0/8 in the Randomized PGE-1 group, and 3/9 in the placebo group (including one death). This was statistically significant (P < .05, chi square).

## Drug Dosage

Prior to the insertion of the balloon catheter. all of the patients received 10,000 bolus units of heparin and intravenous nitroglycerine, as required, as well as sublingual nitrate and beta blockers or intraveneous verapamil.

Prostaglandin E-1 was injected at sixty five nanograms intracoronary before and after the angioplasty procedures. Further, twenty nanograms per kilogram of body weight per minute were then infused intravenously into the patients over a twelve hour period, intravenously while monitoring the patients in an intensive care unit. The dosage was gradually tapered to 0 over the last six hours of this twelve hour period.

The placebo composition was administered to the patients in the placebo group much in the same manner as the prostaglandin E-1 was administered to the patients in the drug group. Finally, the proper composition known by the pharmacist was administered to each of the patients in the open label group.

## EXAMPLE III

2D echocardiogram testing was performed with each of the patients identified in Example II. A 2D echocardiogram is placed over the heart during the occlusion and an LV function change was measured simultaneously.

The 2D echocardiogram was re-measured at three and six month intervals after the angioplasty procedure in order to determine the effects of the prostaglandin composition and the angioplasty procedure itself on left ventrical function, both globally and regionally. Also, duration of reactive hypermia was measured after ballon inflation along with a gradient as an approximation of coronary blood flow.

## Method of Measuring Coronary Blood Flow

The method of Vogel, et al was used to measure coronary blood flow (Application of Digital Techniques to Selective Coronary Arteriography. Use of Myocardial Contrast Appearance to Measure Coronary Flow Reserve, American Heart Journal, Volume CVII, Number 1, January 1984).

The prostaglandin compound is used as a hyperemia inducing substance, instead of using contrast. Beyond that, the method and equipment are identical. Digital processing of the 35 mm. cine films was

12

digitized. Gated interval differencing and functional image generation were employed in accordance with the method of Vogel. The digital images were stored in a disk memory and subsequent processing was performed on a mini computer.

A single intensity and color modulated functional image is generated in five colors. Each corresponds to one of the five post-contrast or prostaglandin injection cardiac cycles analyzed and are used to display the cycle in which contrast medium appeared in each pixel.

In accordance with the technique described by Vogel, supra, 256 intensity levels were used to depict the relative amount of the increase in contrast medium that occured in each pixel during its appearance in a cycle. This form of dual-function arteriographic image has been termed "contrast medium appearance picture". This is coupled with the myocardial contrast appearance time, i.e. the time from the onset of contrast injection to its appearance within the region of myocardium, and this was then used as an index of regional coronary blood flow.

In order to measure intracoronary blood flow before and after prostaglandin administration, digital radiographic assessment of contrast medium appearance time distal to the targeted stenosed coronary artery was used. Measurements were made for prostaglandin P-1 under hyperemic conditions, i.e. 65 nanograms of intracoronary prostaglandin E-1 after 45 seconds, versus a control, and nonhyperemic conditions, i.e. intracoronary placebo (5 cc. of absolute alcohol), to measure myocardial blood flow before and immediately after the coronary angioplasty. The accepted method of the reciprocal of the coronary flow ratio was used to determine myocardial blood flow, substituting, however, intracoronary prostaglandin E-1 for contrast as the hyperemia inducing agent. Neither the intracoronary prostaglandin E-1 nor placebo elicited any angina, dysrhythmia, or significant pressure changes. Equivalent dosages for other intracoronary E-1 like prostaglandins are also employed, based on the compound's potency as compared to intracoronary prostaglandin E-1. The intravenous route is preferred for prostaglandin E-1 like prostaglandin compounds (including PGE-12, its salts, esters, andamids). However, other systemic routes of administration (e.g. oral, parenteral, intra-arterial or dermal) may also be employed as long as the dosage used achieves the same blood level of drug as the intravenous route.

As summarized by Vogel in The Radiographic Assessment of Coronary Blood Flow Parameters, Circulation, Volume LXXII, Number 3, September 1985, this imaging technique to measure coronary blood flow is performed rapidly during cardiac catheterization. However, atrial pacing, ECG synchronized power injection of contrast media, digital radiographic equipment capable of direct digital storage and fixed patient positioning were added to the routine technique and did not result in any problems.

## Correlative Results

Seventeen doubly blinded patients did provide data which allowed for complete analysis. Nine patients were given the placebo hand-injected, bolus, intracoronary before and after the angioplasty procedure, and the myocardial blood flow was measured as described above.

Under double blinded conditions, the placebo given was the 4 or 5 cc. of normal saline, and this same vehicle was used for the prostaglandin E-1. In this group of nine, there was one death in 24 hours and two episodes of prolonged angina documented by ECG change. This did not appear in the patients given the 65 nanograms of intracoronary prostaglandin E-1 hand-injected, bolus, in the doubly blinded drug group.

The percent stenosis of the target vessel in the placebo and drug groups were similar, being 80 percent mean pre-angioplasty and 25 percent mean post-angioplasty, as measured by standard visual criteria. The patients receiving the drug in the open label group had similar degrees of stenosis in their coronary arteries prior to angioplasty. The incidence of unstable angina, antecedent myocardial infarction, or post-infarction angina, was similar in all three groups, namely, 33 percent in the placebo group, 25 percent in the drug group, and 29 percent in the open label group. Regarding recent myocardial infarction, there was one patient with unstable angina post-infarction in the drug group, none in the placebo group, and two in the open label group.

When comparing the placebo group with the intracoronary drug group, the nine patients receiving the intracoronary placebo injection, pre-coronary angioplasty, showed a reciprocal of the coronary flow ratio of 1.04 ±0.13 standard error of the mean. Eight patients in the drug group, pre-coronary angioplasty, and the seven patients in the open label group, pre-coronary angioplasty, showed a coronary flow ratio of 11 ±0.17, and the difference between the placebo and drug group was not significant pre-angioplasty. It should be noted that despite the tight flow limiting stenoses in the stenosed coronary arteries, intracoronary prostaglandin E-1 did augment flow slightly, but this was not significant because of the flow limiting stenosis.

The nine patients in the placebo group, post-angioplasty, had a coronary flow ratio of 1.54 ±.12, which is higher than the pre-coronary angioplasty flow. In the drug group and the open label group. the coronary angioplasty elicited an intense hyperemia, which yielded a coronary flow value of 2.31 ±0.34 with the P-value of points less than .001 using a student two tailed T-test. In the open label group. the duration of the hyperemia was tested with two and five minute repetitions of the coronary flow value and the hyperemia was still much above a baseline flow. In two patients in the open label group. the comparison of the hyperemic response of intracoronary prostaglandin E-1 was 22 percent higher than judged by the coronary flow value.

Two-dimensional echocardiography was performed immediately after the angioplasty and repeated at least once within two days prior to the patient being discharged. i.e. whenever clinically feasible. The echocardiography did show. in the placebo group, no significant increase in regional ventricular function. In the doubly blinded drug group. three of eight patients did show increase in regional function as judged by independent analysis by a blinded investigator. via 2D echocardiographic criteria. In the open label group. two of seven patients did show dramatic increase in regional LV function. However. one patient did have unstable angina prior to the angioplasty.

Conclusions

Based on the foregoing analysis. it was concluded that the intracoronary prostaglandin E-1 and the intravenous prostaglandin E-1 in the above described dosages was safe and created no serious side effects. Moreover, the prostaglandin compounds. when used as described herein, elicit an intense hyperemia, at least doubling coronary blood flow directly. This effect appears to be prolonged without inducing any hemodynamic complications. such as hypotension.

The vasodilating and hyperemic effects of prostaglandin E-1 appears to be a direct result of its antispasm effect in the area where the plaque is ruptured by balloon dilation. Angiography reveals that there appears to be, in almost all instances of angioplasty, a subintimal disruption and which is the technique by which the plaque is fractured during the angioplasty procedure and hence the flow obstruction removed.

Thus. it was concluded that in unstable angina the prostaglandin E-1 and the other prostaglandin compounds do inhibit platelet desposition at the site of atheroma fracturing during angioplasty. Consequently, embolization of microemboli platelets in a downstream position is not noted. As a result, and as could be anticipated, prolonged angina or abrupt closure was not observed in the drug group, as it was observed in the placebo group.

It was postulated that it is really impossible to determine the appearance and condition of coronary vessels in a post-angioplasty procedure condition by angiographic criteria. Under pathologic studies previously performed, and under angioscopy, a roughing and tearing of the intima and media is almost always observed. Consequently, the angiogram therefore underestimates the amount of damage to a vessel which is subjected to an angioplasty procedure. The site of the procedure is unable to synthesize local prostaglandins. Thus, the thromboxane that is released by the platelets is unopposed which results in spasm or thrombus. The exogenous prostaglandin E-1 and the other prostaglandin compounds encompassed by the present invention delivered in sufficient dosages in accordance with the present invention have been shown to overcome these effects.

It was also concluded that the hyperemic or increased blood flow state induced by the intracoronary prostaglandin was prolonged more than with intravenous nitroglycerine. Thus, it was also concluded that it is desirable. in most cases, to continue the intravenous prostaglandin E-1 for at least 12 hours post-angioplasty and post-intracoronary injection to obtain the most beneficial effects of the prostaglandin compound.

Based on the foregoing, it was concluded that the prostaglandin compound exhibits substantial antispasmotic effects, and antithrombotic and antiplatelet effects, as abrupt occlusion and thrombosis were not observed in the drug group, but were observed in the placebo group. The prostaglandin compound does exhibit a myocyte protection effect, both cellular and as an inhibitor of platelet aggregation and deposition.

EXAMPLE IV

Eighty patients were evaluated in a double blind study in order to determine whether an intravenous infusion of prostaglandin E-1 produced the desired salutary effects after a coronary angioplasty procedure. Twenty to sixty nanograms per kilogram per minute of prostaglandin E-1 (test patients) versus a normal

14

saline placebo (placebo patients) was infused into certain of the test patients for a period of twelve hours after the angioplasty procedure. Further, one hundred and thirty nanograms bolus of prostaglandin E-1 was administered in an intracoronary procedure during the angioplasty procedure. All of the patients were prepared prior to angioplasty in accordance with the typical procedures with asprin, dipyridamole, heparin, nitrites and nifedipine and/or verapamil. Moreover, administration of these latter drugs continued after the angioplasty procedure.

## Abrupt Occlusions

The prostaglandin E-1 produced a reduction in a number of eposides of abrupt occulsions with three occuring in forty patients which did not recieve the prostaglandin E-1 and none in forty patients which did receive the prostaglandin E-1 ($P < .05$ chi square). All patients with dissection and/or prolonged spasm had repeat angiograms within seven days. With dissection during the angioplasty procedure, prostaglandin E-1 inhibited prolonged spasms (2/5 for those patients not receiving the prostaglandin and 0/6 for those patients receiving the prostaglandin) and restenosis despite systemic heparinization for two to seven days after the angioplasty procedure (2/2 for patients not receiving the prostaglandin E-1 versus 0/6 for patients receiving the prostaglandin E-1).

These eighty patients were followed clinically for six months. Four patients returned for repeat angioplasty because of angina pectoris and coronary restenosis. Restenosis was defined as greater than 50% luminal diameter reduction in a coronary artery previously dialated to a residual luminal diameter of less than 50%. All four restenosis patients were in the placebo group. Restenosis did not occur in six months in the drug group. (0 of 40 vs. 4 of 40, $P < .03$).

## Side Effects

Adverse effects of prostaglandin E-1 infusion at this stage did not occur, primarily because the patients were deliberately volume overloaded. In conclusion, it was determined that a prostaglandin E-1 injection intracoronary followed by intravenous infusion in patients undergoing the angioplasty procedure is quite safe. Prostaglandin E-1 appears to inhibit coronary spasms abrupt occlusion and early restenosis, particularly when dissection is produced by the angioplasty procedure. This salutory clinical effect of prostaglandin E-1 occurs in addition to the effects of standard pharmacutical agents and anti-platelet agents currently used in angioplasty procedures.

One of the unique results which has been found in connection with the present invention is a very substantial reduction in restenosis, as is evident by the foregoing Example III. The problem of restenosis in connection with angioplasty procedure is one which has plagued cardiologists for a long period of time. Moreover, little is known about the causes of restenosis. Nevertheless, it has been observed that there is a very substantial decrease in restenosis when the prostaglandin compound is administered in the ranges described herein.

More specifically, it has been found that the restenosis is decreased substantially as a result of the intra-arterial administration immediately before and/or immediately after the angioplasty procedure and which is followed by the intravenous administration of the prostaglandin, as described above. In the eighty cases tested, as in Example III, only four of the patients demonstrated early restenosis and all four patients were in the placebo group. In other words, all patients who received the prostaglandin compound in accordance with the procedure specified herein suffered no early restenosis. It is preferred to use the same prostaglandin compound for the intravenous administration as was used for the intra-arterial administration, although this is not absolutely necessary.

Although it is not certain, it is theorized that the post-angioplasty procedure intravenous infusion in combination with the intracoronary administration leads to the significant results and substantial reduction of restenosis. It is believed that the infusion during the last nine to fifteen hours and preferably the last twelve hours affects the platelet deposition and platelet aggregation. Thus, by reducing the platelet deposition and aggregation, previously encountered problems of early restenosis have been reduced, if not fully eliminated.

## EXAMPLE V

In three individual patients with acute myocardial infarction, streptokinase was given intravenously prior to transluminal angioplasty procedures. This was followed within 24 hours, 23 hours, and 3 hours respectively for each of the three patients by intracoronary injection of prostaglandin E-1, and in one patient, a mixture of streptokinase and prostaglandin E-1. After the angioplasty procedure, the prostaglandin E-1 was administered intravenously for 12 hours.

In the first patient, the streptokinase was given 24 hours prior to the angioplasty procedure and followed by spasm and continued angina requiring emergency transfer for angioplasty and intracoronary and intravenous prostaglandin therapy. In patient number two, with similar circumstances, 12 hours following thrombolysis with intravenous streptokinase, spasm and post infarction angina occurred. This again resulted in emergency ambulance transfer to another hospital for intracornary prostaglandin administration and angioplasty followed by intravenous prostaglandin E-1. In the third patient, three hours into an acute myocardial infarction, prostagalndin E-1 in an amount of 130 nanograms plus streptokinase in an amount of 100,000 units was injected intracornary with thrombolysis. This was followed by successful angioplasty also followed by intravenous prostaglandin E-1 perfusion for 12 hours.

In all three cases of the above identified patients, there was salvage of the left ventricular myocardium which was proven by improvement two dimensional echocardiography parameters. The echocardiography was taken immediately before and immediately after the angioplasty procedure as well as 24 hours after the angioplasty procedure and the prostaglandin E-1 infusion.

The study of the above identified patients demonstrates effectively the use of the thrombolysis with intracornary prostaglandin and streptokinase for treatment of acute myocardial infarctions. The left ventricular recovery is enhanced with the post-thrombolysis and angioplasty which inhibits early reocclusion of the infarct vessel as well as the inhibition of cornary spasm, platelet deposition and rethrombosis.

## EXAMPLE VI

In one patient, prostaglandin E-1 in an amount of 130 nanograms was mixed with 25,000 units of streptokinase and injected intracoronary to dissolve a clot in the coronary artery which caused the acute myocardial infarction. This was followed with a balloon angioplasty to relieve the original 95% stenosis which is generally present in about 90% of the patients. Thereafter, digital subtraction angiography flow studies were performed and dispite the lysis of the clot, there remained severe tissue damage, tissue edema of the muscle cell itself, free radical scavengers, and other metabolic processes leading to cell death.

The prostaglandin in combination with the thrombolytic agent, such as the streptokinase, was found to inhibit cell necrosis, and the no reflow phenomenon by digital subtraction angiography techniques of Vogel.

When the prostaglandin E-1 and thrombolytic agent was given to patients in rural areas over a 30 minute period, they were then transferred to a hospital having modern coronary care facilities within about a 4 hour period. In most cases, it had been found that any blood clot had been dissolved by the thrombolytic agent. However, in about 90% of the cases, the patients were in jeapardy since these clots formed in a ruptured denuded area of significant stenosis in the coronary artery. As a result, the heart muscle suffered edema, hemorrhage, and the beginning phases of cell death thereby endangering the lives of the patients.

In this case where the patient's life was in danger, he was then immediately taken to a facility and injected with 10 to 50,000 units of streptokinase mixed with 130 grams of prostaglandin E-1 as a bolus injection into the infarct coronary artery. This injection was found to clear the microemboli or very small clots down stream from the dissolved clot. At this point in time, a wire and balloon are introduced into the residual lesion and an emergency angioplasty is performed to restore blood flow to the area of the infarcted myocardium.

At the time of the emergency angioplasty procedure, 40 nanograms per kilogram per minute of intravenous infusion was administered to the patients. This amount was given for a 12 hour period to inhibit the effects of the no reflow phenomenon which has been found to be present in essentially all cases of acute myocardial infraction.

16

## RELATED APPLICATION

This application is a continuation-in-part patent application of our co-pending patent application Serial No. 848,233, filed April 4, 1986, entitled "METHOD AND COMPOSITIONS FOR REDUCING DYSFUNCTION IN ANGIOPLASTY PROCEDURES" which is, in turn, a continuation-in-part of our co-pending patent application Serial No. 784,160, filed October 4, 1985, entitled METHOD FOR REDUCING DYSFUNCTION IN ANGIOPLASTY PROCEDURES.

## Claims

1 A method for reducing restenosis and occlusion and clot generation in human blood vessels, said method comprising administering a thrombolytic agent in a pharmacological amount to a patient having an unstable ischemic episode or in which a blood vessel has been insulted, and administering to the patient a pharmacological amount of a prostaglandin compound to provide cell protection and antithrombotic effects and antiplatelet effects and antispasmotic effects.

2 The method of Claim 1 further characterized in that the method comprises administering the prostaglandin compound and the thrombolytic agent directly to an insulted blood vessel.

3 The method of Claim 2 further characterized in that the method comprises administering a mixture of the thrombolytic agent and prostaglandin compound intravenously and intra-arterially.

4 The method of Claim 2 further characterized in that the prostaglandin compound is introduced into the artery in an amount which is substantially greater than the amount of prostaglandin which would be generated by such artery if in a normal condition and insulted and the thromboloytic agent is administered in an amount of about 50,000 units to about 3,000,000 units.

5 The method of Claim 4 further characterized in that said method comprises introducing into the artery in which such procedure occurs the intra-arterial prostaglandin compound in an amount to produce an efficacy approximately equivalent to that produced by the administration of about 25 nanograms to about 3,000 nanograms of prostaglandin E-1

6 The method of Claim 5 further characterized in that said method comprises introducing directly into the artery in which an angioplasty procedure occurs prior to or during the angioplasty procedure, an initial amount of the intra-arterial prostaglandin compound and intraveneously administering the prostaglandin compound for a period of about 9 hours to about 48 hours after said angioplasty procedure.

7 A combination of components effective for reducing restenosis and occlusion and clot generation in human blood vessels which have been insulted, and which components are adapted for at least bolus administration, said combination comprising a prostaglandin compound which in a phamacological amount provides cell protection and antithrombotic effects, antiplatelet effects and antispasmotic effects, and a thrombolytic agent which when applied in a pharmacological amount will reduce clot re-formation.

8 The composition of Claim 7 further characterized in that the prostaglandin compound is present in an amount necessary tc produce an efficacy approximately equivalent to the efficacy produced by the administration of protaglandin E-1 in a range of about 12 nanograms to about 200 nanograms.

9 A composition of Claim 7 for intravenous administration to a human being which is effective to reduce dysfunction in angioplasty procedures in that human being, said a prostaglandin compound provided for intravenous administration over a selected time period at a relatively constant rate and in an amount to produce an efficacy approximately equivalent to that produced by the administration of prostaglandin E-1 in a range of about 10 nanograms to about 100 nanograms per kilogram of body weight per minute, and the thrombolytic agent is present in the composition in an amount of about 50,000 units to about 3,000,000 units.

10 A method for reducing restenosis and occlusion and clot generation in human blood vessels which have been insulted or which suffer dysfunction as a result of an ischemic episode, said method comprising administering a thrombolytic agent in a pharmacological amount to a patient, and administering to the patient an initial pharmacological amount of an intra-arterial prostaglandin compound to provide cell protection and antithrombotic effects and antiplatelet effects and antispasmotic effects, and further intravenously administering additional prostaglandin compound overa period ranging from about 9 hours to about 48 hours to reduce early restenosis.

11 The method of Claim 10 further characterized in that the method comprises administering the prostaglandin compound directly to an insulted blood vessel.

12 The method of Claim 11 further characterized in that the initial amount of prostaglandin compound is introduced into an artery in an amount which is substantially greater than the amount of prostaglandin which would be generated by such artery if in a normal condition and insulted. and the thrombolytic agent is administered in an amount of about 50,000 units to about 3,000,000 units.

13. A packaged combination of components effective for reducing blood vessel dysfunction resulting from unstable anginal ischemia or as a result of insult by mechanical means, said combination comprising:

a) a first container having a thrombolytic agent in a pharmacological amount to substantially reduce the potential for clot formation as a result of unstable anginal ischemia or insult from mechanical means.

b) a second container having a prostaglandin compound in an amount necessary to produce an efficacy in a range approximately equivalent to the efficacy produced by at least a minimum of about 25 nanograms of prostaglandin E-1 to a maximum amount of 3,000 nanograms of prostaglandin E-1 by bolus injection into an artery of a patient, and

b) a third container having a prostaglandin compound for intravenous administration after the bolus administration of the first and second containers and which intravenous administration takes place for a selected time period after or during the ischemia or the insult by mechanical means and at a rate in accordance with the body weight of the patient. the prostaglandin compound in the third container being present in an amount to produce an efficacy approximately equivalent to the efficacy produced by prostaglandin E-1 when administratered to a patient in an amount of about 10 nanograms to about 100 nanograms per kilogram of body weight per minute.